(19) Europäisches Patentamt
European Patent Office
Office européen des brevets

(11) Veröffentlichungsnummer: **0 173 975 B1**

# (12) EUROPÄISCHE PATENTSCHRIFT

(45) Veröffentlichungstag der Patentschrift: 07.12.88

(51) Int. Cl.⁴: **C 07 D 249/08**, C 07 D 231/12, C 07 D 233/60, C 07 D 235/06

(21) Anmeldenummer: 85110923.1

(22) Anmeldetag: 30.08.85

## ERRATUM

| DIE TEXTSTELLE: TEXT PUBLISHED: LE PASSAGE SUIVANT: | (SEITE, SPALTE, ZEILE) (PAGE, COLUMN, LINE) (PAGE, COLONNE, LIGNE) | | | LAUTET BERICHTIGT: SHOULD READ: DEVRAIT ETRE LU: |
|---|---|---|---|---|
| einer hetrocyclischen | 2 | 1 | 6 | einer heterocyclischen |
| Alkoxy($C_1$-$C_4$), Phenyl | 2 | 1 | 64 | Alkoxy($C_1$-$C_2$), Alkyl($C_1$-$C_4$)Phenyl |
| einen Triazol-, Benzimidazol- | 3 | 4 | 38 | einen Triazol-, Pyrazol Benzimi-dazol |

Tag der Entscheidung über die Berichtigung / Date of decision on rectification: / Date de décision portant sur modification: 17.04.89

Ausgabe- und Veröffentlichungstag: / Issue and publication date: / Date d'edition et de publication: 09.07.89

Patbl.Nr. / EPB no: / Bull. no: 89/29

(19) Europäisches Patentamt
European Patent Office
Office européen des brevets

(11) Veröffentlichungsnummer: **0 173 975 B1**

# (12) EUROPÄISCHE PATENTSCHRIFT

(45) Veröffentlichungstag der Patentschrift: **07.12.88**

(51) Int. Cl.⁴: **C 07 D 249/08**, C 07 D 231/12, C 07 D 233/60, C 07 D 235/06

(21) Anmeldenummer: **85110923.1**

(22) Anmeldetag: **30.08.85**

(54) **Verfahren zur Herstellung von Keten-0, N-acetalen.**

(30) Priorität: **05.09.84 DE 3432579**

(43) Veröffentlichungstag der Anmeldung: **12.03.86 Patentblatt 86/11**

(45) Bekanntmachung des Hinweises auf die Patenterteilung: **07.12.88 Patentblatt 88/49**

(84) Benannte Vertragsstaaten: **AT BE CH DE FR GB IT LI NL SE**

(56) Entgegenhaltungen:
**EP-A- 0 056 125**
**EP-A- 0 110 116**

**HOUBEN-WEYL "Methoden der Organischen Chemie", 4. Auflage, Band VII/4, 1968, Sauerstoffverbindungen II, Georg Thieme Verlag, Stuttgart; D. BORRMANN "Methoden zur Herstellung und Umwandlung von Ketenen, dimeren Ketenen, Keten-Polymeren, Kohlensuboxid, Thioketenen, Kohlensubsulfid, Keten-iminen, Keten-acetalen und deren Derivaten"**

(73) Patentinhaber: **BASF Aktiengesellschaft, Carl-Bosch-Strasse 38, D-6700 Ludwigshafen (DE)**

(72) Erfinder: **Ruland, Alfred, Dr., Schriesheimer Strasse 11, D-6945 Hirschberg (DE)**
Erfinder: **Reuther, Wolfgang, Dr., Am Pferchelhang 16, D-Heidelberg (DE)**

**EP 0 173 975 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von Keten-O,N-acetalen durch Umsetzung der entsprechenden Keten-O,O-acetale mit einer hetrocyclischen Stickstoffverbindung in Gegenwart saurer Katalysatoren.

Keten-O,N-acetale und ihre Anwendung als Fungizide sind bekannt (EP-56 125). Zu ihrer Herstellung sind zwei Verfahren bekannt.

So lassen sie sich z.B. durch Zersetzung der entsprechenden Phenylsulfonate (EP-56 125) bei 10 bis 100 °C in Gegenwart eines Lösungsmittels und eines basischen Katalysators gewinnen. Dieses Herstellungsverfahren hat den Nachteil, dass zum einen die benötigten Vorstufen nur schwierig und teuer zugänglich sind, und dass zum anderen die Keten-O,N-acetale als Mischung der entsprechenden E/Z-Isomere anfallen. Für den Fall, dass nur eines der beiden Isomeren von Interesse ist, müssen die Isomeren getrennt werden, und es entsteht ein Zwangsanfall an unerwünschten Isomeren.

Beim zweiten bekannten Verfahren (EP-110 116) werden Keten-O,O-acetale als Ausgangsverbindungen eingesetzt. Dabei lassen sich die oben genannten Schwierigkeiten zwar vermeiden, das Verfahren hat jedoch den Nachteil, dass sehr lange und damit unwirtschaftliche Reaktionszeiten benötigt werden. Darüber hinaus sind Umsätze und Ausbeuten schlecht.

Es wurde nun überraschend gefunden, dass man Keten-O,N-acetale der Formel I

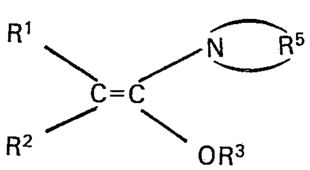

I

in der

$R^1$ einen tertiären Alkylrest mit 4 bis 6 C-Atomen,

$R^2$ Wasserstoff,

$R^3$ einen gegebenenfalls durch Halogen, Alkoxy, Alkyl, Phenyl, Phenoxy, Cyano, Nitro, Trifluormethyl, substituierten Phenylrest und 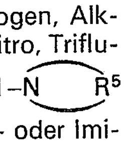 einen Triazol-, Pyrazol-, Benzimidazol- oder Imidazolrest bedeutet, ohne die genannten Nachteile erhält, wenn man ein Keten-O,O-acetal der Formel II

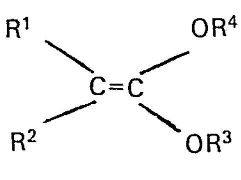

II

in der

$R^1$, $R^2$ und $R^3$ die oben genannten Bedeutungen haben und $R^4$ einen gegebenenfalls durch Halogen, Alkoxy($C_1 - C_4$), Phenyl oder Phenoxy, Cyano, Nitro, Trifluormethyl substituierten Phenylrest bedeutet, bei erhöhter Temperatur mit einer Verbindung der Formel

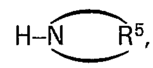

in der 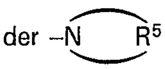 die oben genannten Bedeutungen hat, in Gegenwart eines sauren Katalysators umsetzt.

$R^1$ bedeutet beispielsweise einen tertiär-Butylrest,

$R^3$ und $R^4$ bedeutet beispielsweise einen durch Halogen (F, Cl, Br), Alkoxy mit 1 bis 2 C-Atomen (Methoxy), Alkyl mit 1 bis 4 C-Atomen (Methyl, Ethyl, Propyl, i-Propyl, Butyl, sek.-Butyl, tert.-Butyl, i-Butyl) substituierten Phenylrest, wobei einzelne Reste einfach oder mehrfach (2- bis 3-fach) oder verschiedene Reste gleichzeitig als Substituenten auftreten, z.B. 4-Chlorphenyl, 3,4-Dichlorphenyl, 4-Chlor- 3-bromphenyl, 4-Methyl- 3-chlorphenyl, 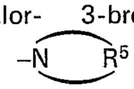 bedeutet beispielsweise 1-(1,2,4)-Triazolyl, 1-(1,2,3)-Triazolyl.

Erhöhte Temperatur bedeutet beispielsweise eine Temperatur höher als 80 °C, insbesondere 100 bis 250 °C, z.B. 110 bis 170 °C.

Als saure Katalysatoren eignen sich organische Sulfonsäuren, z.B. para-Toluolsulfonsäure oder Dodecylbenzolsulfonsäure, ferner Bortrifluorid-Komplexe, z.B. $BF_3$-Etherat, $BF_3$-Essigsäure oder $BF_3$-Komplexe der entsprechenden cyclischen Aminoverbindung 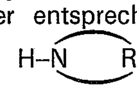, oder anorganische Säuren wie Salzsäure, Bromwasserstoffsäure, Iodwasserstoffsäure, Phosphorsäure oder Schwefelsäure.

Für die Umsetzung werden etwa äquimolare Mengen an Keten-O,O-acetal und heterocyclischer Stickstoffverbindung verwendet. Man kann jedoch auch einen Überschuss an einem Reaktionsteilnehmer, beispielsweise an dem billigeren Reaktionsteilnehmer, z.B. einem Überschuss bis 50%, insbesondere bis zu 30%, verwenden. Die für die Reaktion notwendige Menge an saurem Katalysator ist sehr gering. Sie beträgt beispielsweise 0,1 bis 5% (Gewichtsprozent, bezogen auf das Gewicht der Ausgangsprodukte), insbesondere 0,5 bis 2%, z.B. 1%.

Die schnelle und quantitative Umsetzung der oben genannten Keten-O,O-acetale zu den entsprechenden Keten-O,N-acetalen der Formel I in Gegenwart von Säuren als Katalysatoren ist insofern überraschend, als man zunächst eine Zersetzung der Keten-O,O-acetale erwarten würde, die bekanntlich extrem säureempfindlich sind. Überraschend ist ferner, dass auch bei saurer Katalyse kein Fortschreiten der Reaktion über die O,N-Acetale hinaus zu Keten-N,N-acetalen beobachtet, und der stereoselektive Verlauf nicht negativ beeinflusst wird. Ein weiterer grosser Vorteil des neuen Verfahrens besteht in der Möglichkeit, die Reaktionstemperatur deutlich abzusenken. So kann z.B. bei der Umsetzung von 1,1-Bis(4-phenyl-phenoxy)-3,3-dimethyl-buten-1 mit Triazol

die Reaktionszeit von 15 auf 1 bis 2 Stunden und die Reaktionstemperatur von 200 auf 160 °C gesenkt werden.

Das erfindungsgemässe Verfahren wird z.B. in Anwesenheit von Lösungsmitteln und vorzugsweise in Abwesenheit von Lösungsmitteln bei Normaldruck durchgeführt. Bei der Umsetzung geht der Rest $-OR^4$ in das entsprechende Phenolderivat $HOR^4$ über. Im Laufe der Umsetzung entstehen steigende Mengen an diesem Phenolderivat. Es stört die Umsetzung nicht und muss daher nicht laufend aus der Umsetzungsmischung entfernt werden.

Nach Beendigung der Umsetzung kann das entstandene Phenolderivat in üblicher Weise, beispielsweise durch Überführung in das entsprechende wasserlösliche Alkaliphenolat, von dem Endprodukt oder seiner Lösung in einem organischen Lösungsmittel abgetrennt werden.

Die Überführung in das Alkaliphenolat erfolgt üblicherweise mit einem wässrigen basischen Extraktionsmittel, z.B. Natronlauge oder Kalilauge, vorzugsweise in Konzentrationen von 5 bis 30% (Gewichtsprozent) in Wasser aus der organischen Lösung des Endproduktes.

Als organische Lösungsmittel kommen aliphatische Kohlenwasserstoffe, Ether oder Ester sowie aromatische Kohlenwasserstoffe wie Toluol oder o,m,p-Xylol in Betracht. Das Endprodukt kann aber auch als quartäres Ammoniumsalz, vorzugsweise mit der wässrigen Lösung einer starken anorganischen Säure, wie Chlorwasserstoffsäure, Bromwasserstoffsäure, Schwefelsäure, Salpeter- oder Perchlorsäure aus der organischen Phase extrahiert werden. Nach Neutralisation der Säure kann das freie Keten-O,N-acetal aus der wässrigen Phase abgetrennt werden.

Die als Ausgangsprodukte benötigten Keten-O,O-acetale der Formel II sind bekannt (EP-37 164) und lassen sich nach bekannten Verfahren herstellen (EP-37 164).

Beispiel 1

Herstellung von Z-1-(1,2,4-Triazol-1-yl)-1-(4-phenylphenoxy)-3,3-dimethyl-buten-1

210 g (0,5 Mol) 1,1-Bis(4-phenylphenoxy)-3,3-dimethylbuten-1, 51,7 g (0,75 Mol) 1,2,4-Triazol und 2 g Dodecylbenzolsulfonsäure werden 2 Stunden unter kräftigem Rühren auf 160 °C erhitzt. Danach ist keine Ausgangsverbindung mehr nachweisbar.

Ausbeute an Keten-O,N-acetal: 67% d.Th., bestimmt mit Hochdruckflüssigkeitschromatographie (HPLC).

Vergleichsbeispiel gemäss EP-110 116

210 g (0,5 Mol) 1,1-Bis(4-phenylphenoxy)-3,3-dimethylbuten-1 und 51,7 g (0,75 Mol) 1,2,4-Triazol werden auf 200 °C erhitzt. Nach einer Reaktionszeit von 15 Stunden wird die Reaktion abgebrochen.

Ausbeute an Keten-O,N-acetal: 47% d.Th., bestimmt mit HPLC.

Beispiel 2

Herstellung von Z-1-(1,2,4-Triazol-1-yl)-1-(4-chlorphenoxy)-3,3-dimethylbuten-1

227 g (1 Mol) 1,1-Bis(4-chlorphenoxy)-3,3-dimethylbuten-1 und 131 g (1,25 Mol) 1,2,4-Triazoliumhydrochlorid werden 1 Stunde auf 170 °C erhitzt. Danach ist keine Ausgangsverbindung mehr nachweisbar.

Ausbeute an Keten-O,N-acetal: 75% (d.Th., bestimmt mit HPLC).

Vergleichsbeispiel gemäss EP-110 116

337 g (1 Mol) 1,1-Bis(4-chlorphenoxy)-3,3-dimethylbuten-1 und 86,25 g (1,25 Mol) 1,2,4-Triazol werden 5 Stunden auf 190 °C erhitzt. Danach ist keine Ausgangsverbindung mehr nachweisbar.

Ausbeute an Keten-O,N-acetal: 55% (d.Th., bestimmt mit HPLC).

## Patentansprüche

1. Verfahren zur Herstellung von Keten-O,N-acetalen der Formel I

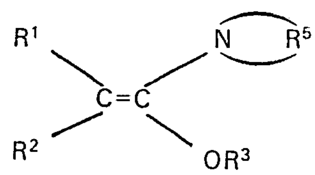

I

in der
$R^1$ einen tert. Alkylrest mit 4 bis 6 C-Atomen,
$R^2$ Wasserstoff,
$R^3$ einen gegebenenfalls durch Halogen, Alkoxy, Alkyl, Phenyl, Phenoxy, Cyano, Nitro, Trifluormethyl substituierten Phenylrest und 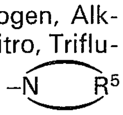 einen Triazol-, Benzimidazol- oder Imidazolrest bedeutet, durch Umsetzung eines Keten-O,O-acetals der Formel II

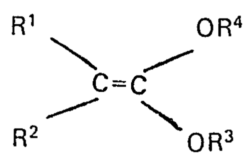

II

in der $R^1$, $R^2$ und $R^3$ die oben genannten Bedeutungen haben und $R^4$ einen gegebenenfalls durch Halogen, Alkoxy, Alkyl, Phenyl oder Phenoxy, Cyano, Nitro, Trifluormethyl substituierten Phenylrest bedeutet, bei erhöhter Temperatur mit einer Verbindung der Formel

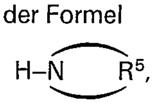,

in der 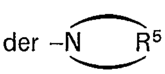 die oben genannten Bedeutungen hat, dadurch gekennzeichnet, dass man die Umsetzung in Gegenwart eines sauren Katalysators durchführt.

2. Verfahren nach Anspruch 1, dadurch gekennzeichnet, dass man die Umsetzung bei einer Temperatur von 100 bis 250 °C durchführt.

3. Verfahren nach Anspruch 1, dadurch gekennzeichnet, dass man die Umsetzung in der Schmelze der Ausgangsprodukte der Formel II und der Verbindung H–N $R^5$ durchführt.

**Revendications**

1. Procédé de préparation de céton-O,N-acétals de formule I

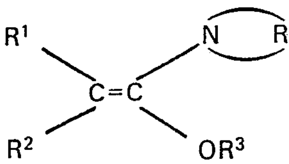

I

dans laquelle

$R^1$ représente un reste alkyle tert. à 4 à 6 atomes C,

$R^2$ hydrogène

$R^3$ un reste phényle, éventuellement substitué par halogène, alcoxy, alkyle, phényle, phénoxy, cyano, nitro, trifluorométhyle, et –N $R^5$ représente un reste triazole, pyrazole, benzimidazole ou imidazole par réaction à température élevée d'un céten-O,O-acétal de formule II

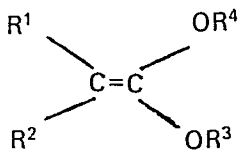

II

dans laquelle $R^1$, $R^2$ et $R^3$ ont les significations sus-indiquées et $R^4$ représente un reste phényle éventuellement substitué par halogène, alcoxy, alkyle, phényle, phénoxy, cyano, nitro, trifluorométhyle, avec un composé de formule H–N $R^5$ dans laquelle –N $R^5$ a les significations sus-indiquées, caractérisé par le fait qu'on effectue la réaction en présence d'un catalyseur acide.

2. Procédé selon la revendication 1, caractérisé par le fait que l'on effectue la réaction à une température de 100 à 250 °C.

3. Procédé selon la revendication 1, caractérisé par le fait que l'on effectue la réaction dans la masse fondue des produits de départ de formule II et du composé H–N $R^5$.

**Claims**

1. A process for preparing a ketene O,N-acetal of the formula I

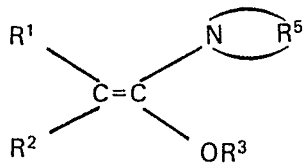

I

where

$R^1$ is tertiary alkyl of from 4 to 6 carbon atoms,

$R^2$ is hydrogen,

$R^3$ is unsubstituted or halogen-, alkoxy-, alkyl-, phenyl-, phenoxy-, cyano-, nitro- or trifluoromethyl-substituted phenyl and –N $R^5$ is triazolyl, pyrazolyl, benzimidazolyl or imidazolyl, by reacting a ketene O,O-acetal of the formula II

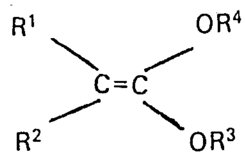

II

where $R^1$, $R^2$ and $R^3$ are each as defined above and $R^4$ is unsubstituted or halogen-, alkoxy-, alkyl-, phenyl-, phenoxy-, cyano-, nitro- or trifluormethyl-substituted phenyl, at elevated temperatures with a compound of the formula H–N $R^5$, where –N $R^5$ is as defined above, which comprises performing the reaction in the presence of an acidic catalyst.

2. A process as claimed in claim 1, wherein the reaction is carried out at from 100 to 250°C.

3. A process as claimed in claim 1, wherein the reaction is carried out in the melt of the starting material of the formula II and the compound H–N $R^5$.